# EUROPEAN PATENT APPLICATION

(11) **EP 0 723 851 A2**
(43) Date of publication of application: **31.07.1996**
(21) Application number: 96100288.8
(22) Date of filing: 10.01.1996
(51) Int. Cl.: B29C 57/10, B29C 65/20, A61M 39/00

(54) **Total containment connect/disconnect device**

(30) Priority: 18.01.1995 US 373981
(71) Applicant: DENCO, INC., Wilmington, DE 19809 (US)
(72) Inventor: Ivansons, Ivars V., Claymont, DB 19703 (US); Ivansons, Valdis, Wilmington, DE 19810 (US); Dudley, Spencer W.C., Wilmington, DE 19803 (US)
(74) Representative: Wagner, Karl H., Dipl.-Ing.

(57) **Abstract**

A plastic tube section is accurately located in a holder of a total containment connect/disconnect device by mounting a key (22) to the tube section. The lower clamp member of its holder has a groove into which the tube section is inserted. A recess extends across the groove and the key is seated in the recess.

## Description

### Cross-Reference to Related Applications

This application is a continuation-in-part of application Serial No. 290,548, filed August 13, 1994 which in turn is a continuation-in-part of application Serial No. 158,505, filed November 29, 1993 which in turn is a continuation-in-part of Serial No. 139,833, filed October 22, 1993 which in turn is a continuation-in-part of application Serial No. 965,875 filed October 23, 1992, now U.S. Patent No. 5,279,685, which in turn is a continuation-in-part of application Serial No. 764,249 filed September 23, 1991, now U.S. Patent No. 5,209,800, which in turn is a continuation-in-part of application Serial No. 682,977 filed April 10, 1991, now U.S. Patent No. 5,156,701, which in turn is a continuation-in-part of application Serial No. 604,967 filed October 29, 1990, now abandoned, which in turn is a continuation-in-part of application Serial No. 569,855 filed August 20, 1990, now U.S. Patent No. 5,141,592.

### Background of the Invention

The present invention is directed to total containment devices for connecting or disconnecting plastic tubes. Various prior art exists disclosing different approaches for welding or disconnecting plastic tubes. In the total containment system disclosed in the various parent patents and applications a device is used which includes a pair of side by side tubes holders, each of which includes upper and lower clamping members for clamping a tube section therebetween. When two tube sections are joined together in a welding or connect operation it is essential that the ends of the tube sections be properly located with respect to each other to assure that the tube ends are heated and then pressed together in the intended manner.

### Summary of the Invention

An object of this invention is to provide techniques for locating a plastic tube section in a tube holder to facilitate the connect or disconnect procedures in the total containment welding or disconnection of plastic tubes.

In accordance with this invention a key member is fixedly mounted to at least one of the tube sections. The key member is then seated in a correspondingly sized recess in the lower clamp member of its holder to assure the proper location of that tube section.

The key may include a tube enclosing section which fits around the tube with a flat wall connected to the tube enclosing section and extending outwardly thereof. The lower clamping member would have a correspondingly shaped recess at the groove in which the tube would be seated. The recess then receives the key so that the upper surface of the flat wall of the key is coplanar or flush with the upper surface of the lower clamping member.

The tube enclosing section could have an internal wall. Thus a portion of a tube could be inserted into the key and abutting against the internal wall. The key thereby holds the two tube portions together. The internal wall would have an inner surface which is of the same dimension as the inner surface of the tube portion so that a continuous inner surface thereby results.

### Brief Description of the Drawings

Figure 1 is a side elevational view partly in section showing a total containment device in accordance with this invention as used in dialysis;
Figure 2 is a top plan view of a portion of the device shown in Figure 1 with the upper clamp member removed;
Figure 3 is a cross-sectional view taken through Figure 2 along the line 3-3;
Figure 4 is a cross-sectional view taken through Figure 2 along the line 4-4;
Figure 5 is a side elevational view of the holder used in the device used in Figures 1-4;
Figure 6 is a schematic top plan view showing two tubes in a position to be welded or connected in accordance with this invention;
Figure 7 is a view similar to Figure 6 showing the tubes welded together;
Figure 8 is a view similar to Figures 6-7 showing a single tube in its position for being disconnected into two separate tube sections; and
Figure 9 is a view similar to Figure 8 showing the two tube sections disconnected from each other.

### Detailed Description

The present invention relates to refinements in the total containment devices described in the parent patents and applications, the details of which are incorporated herein with respect to each of the parent patents and applications. In general, the device 10 includes a pair of side by side tube holders 12,14 which are schematically shown in Figures 6-9. The holders are used for clamping tube sections 16,18 which would be connected by being welded together as in Figures 6-7 or being disconnected by being separated from each other as in Figures 8-9. In the use of the total containment device a flat seal 20 is formed which is generally horizontal in its wide dimension. The present invention is intended to assure that the proper orientation of the tube results in use of the total containment device. This means in having the seal 20 remain horizontal and in having the end of the tube extend outwardly from the tube holder by the desired distance.

The drawings illustrate the utilization of a key member 22 which accomplishes this location and orientation feature. As shown in the drawings only one key 22 is provided on one of the tube sections 16. If desired the invention may be practiced by having a key on the other tube section 18. The provision of a single key 22, however, serves remarkably well for assuring proper orientation and spacing of the tube sections.

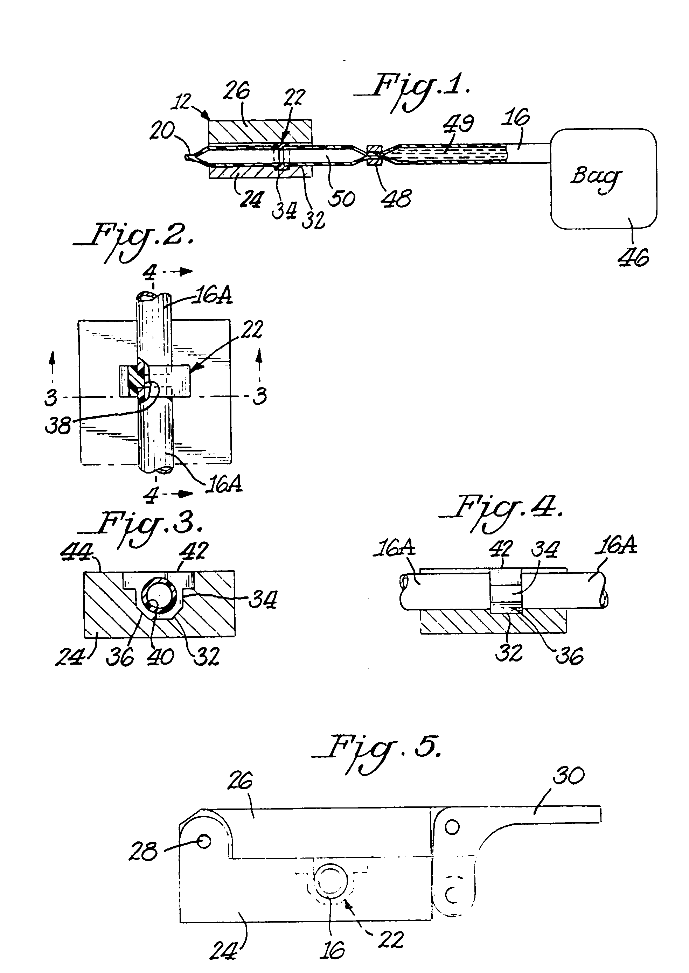

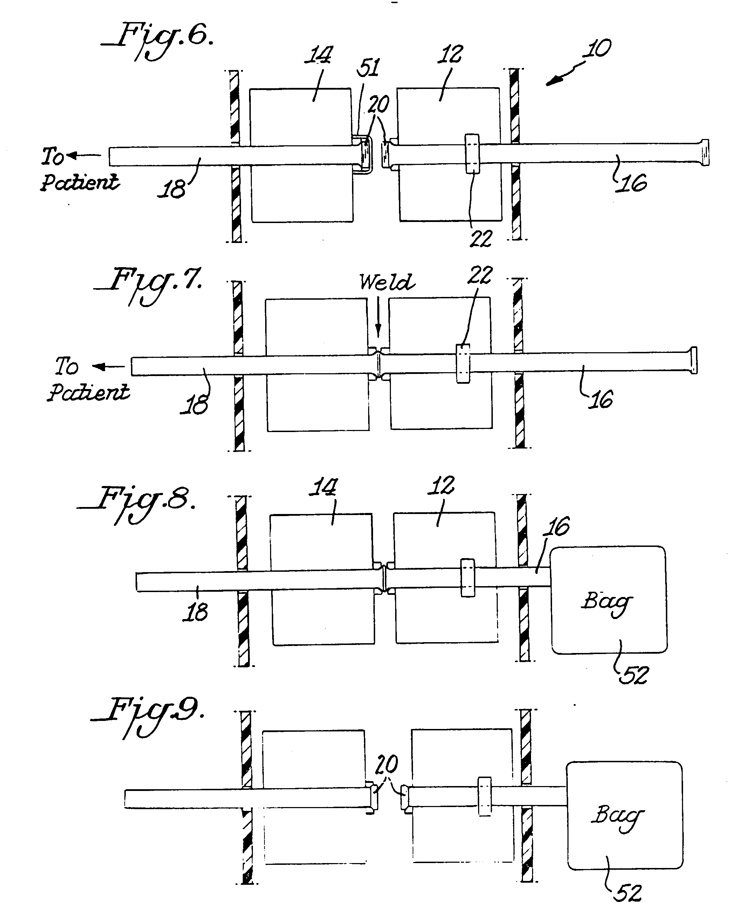

Figures 1-5 illustrate in more detail the holder 12. As shown therein holder 12 includes a lower clamping member 24 and an upper clamping member 26 pivotally mounted thereto at pivot pin 28 so that upper clamping member 26 may move toward and away from lower clamping member 24 to clamp the tube section 16 in position. Handle 30 is utilized for manipulating the upper clamping member 26 in the manner described in the parent patents and applications.

Lower clamping member 24 includes a groove 32 which extends completely longitudinally across the lower clamping member 24 so that its tube section 16 may be mounted in the groove and would be in alignment with the corresponding tube section 18 located in a similar groove of holder 14.

In accordance with this invention a recess 34 is formed transversely across groove 32 at a predetermined distance so that the key 22 may be seated in recess 34 and thereby locate the free end of tube section 16 the desired distance into the space between tube holders 12 and 14. Because of the irregular shape of recess 34 and the corresponding shape of key 22, rotation of tube section 16 in groove 32 is prevented. This assures maintaining the welded seal 20 in a horizontal orientation.

As illustrated in Figures 1-5 and in particular in Figures 2-3, key 22 includes a tube enclosing member 36 which has an inner wall 38 so that two tube portions 16A,16A may be telescoped into tube enclosing member 36 until the tube portions 16A,16A abut against wall 38. Tube portions 16A,16A are sealed to tube enclosing member 36 by any suitable means such as the use of a cyclohexonone seal. This assures that there can be no relative sliding or rotational movement of key 22 with respect to tube section 16. In the preferred practice of this invention the wall 38 has a circular opening with an inner surface of the same dimensions as and thus be flush with the inner surface 40 of tube section 16. This assures unimpeded flow through the tube section 16 even where key 22 is located.

Key 22 also includes a flat elongated top wall 42 which extends outwardly from tube enclosing member 36. The upper surface of wall 42 is flat so as to be flush with the upper surface 44 of lower clamping member 24 as best shown in Figure 3.

Tube enclosing member 36 may be of any shape but preferably includes flat surfaces to fit snugly against the corresponding flat surfaces in recess 34 across groove 32.

Figure 1 illustrates use of the invention for substituting a new dialysis bag for a used bag. Bag 46 would contain a solution such as a dialysate which would be free to flow through tube section 16. When it is intended to connect tube section 16 to a tube 18 leading from a patient such as shown in Figures 6-7 it is first necessary to remove the seals 20 so that the tubes 16 and 18 can be welded together and communicate with each other. In order to prevent any of the fluid or solution from escaping from tube 16, a clamp 48 is applied a suitable distance from the seal 20. See Figure 1. In a practice of the invention key 22 would be located 2.90 cm. from the distal end or seal 20 while clamp 48 would also be located 2.90 cm. from key 22.

As shown in Figure 1 the solution or fluid 49 from bag 46 thus can not pass through clamp 48. Accordingly, a dry chamber 50 is formed in the remaining portion of tube section 16 so as to prevent any fluid spillage when seal 20 is broken.

Key 22 may be made of any suitable material such as rigid PVC. The tubes may also be of any suitable material such as but not limited to conventional PVC tubing.

Figures 6-7 show use of the invention for connecting two tube sections 16 and 18 together. As shown therein each tube section 16,18 would be clamped in its appropriate holder 12,14 with the sealed or welded ends 20,20 disposed toward each other. A moveable locating wall 51 is mounted at seal 20 on holder 14. Wall 51 functions as a stop wall to limit the extent to which tube section 18 may extend beyond its tube holder 14. After tube section 18 is properly positioned in holder 14, wall 51 is removed from contact with the seal 20 at the end of tube section 18 so that each seal 20 of tube sections 16 and 18 are exposed for being heated and thereby facilitate the welding or connection of the tube sections together.

A heated wafer (not shown) would pass through the space between the two sealed ends 20,20 to melt the ends. In the next step of operation which is shown in Figure 7 the melted ends are fused together so that an integral tube results from tube sections 16,18 in the known manner for the total containment device.

Figures 8-9 illustrate the practice of the invention where an integral tube is disconnected to form two separate sections 16,18. As shown in Figure 8, the single tube is disposed across the side by side tube holders 12,14 with key 22 controlling the location of the tube in both of the holders 12,14. A heated wafer would melt the tube in the space between the two tube holders and would form seals 20 which are clearly shown in Figure 9 when the tube holders are separated away from each other. This disconnect procedure would be utilized, for example, to disconnect a used bag 52 so that a new bag 46 could later be connected to tube section 18 leading from the patient as previously described.

The advantages of the present invention include the ability to be able to accurately insert the tubing in the tube holder. Where only one key is used the key would be mounted on the tubing leading from the solution bag. The invention also permits precise square seals to be formed. The welded seals are particularly strong. The distal end is made from dry tubing by the utilization of the clamp 48 as previously described. Clamp 48 would be removed after the connect operation is done. With the practice of the invention there is minimum tubing loss. The invention provides the ability to disconnect exactly at the previous weld because the key controls the location of the tubing. By disconnecting at the previous weld, the tubing loss might be only about 1.0 cm. per day or approximately 2.0 mm. per cycle from the patient's end otherwise the tubing loss would be 10 cm. per day.

By utilizing a key on the tube section 16 there is assurance of having one tube section fixed in its location thus leaving only one variable to be verified for the connect procedure. The key also serves as a means for product identification to assure a user that the solution bag and its tubing are being provided from a proper supplier.

It should be noted that the objects and advantages of the invention may be attained by means of any compatible combination(s) particularly pointed out in the items of the following summary of the invention and the appended claims.

### SUMMARY OF INVENTION

1. In a device for the sterile connect/disconnect of plastic tubing having a pair of side by side tube holders, each of said tube holders having a lower clamp member and an upper clamp member movably connected to said lower clamp member for selective movement toward and away from said lower clamp member to selectively clamp a tube section therebetween with the tube section in each of said holders aligned with each other and extending into a space between said holders, and means for heating the tubes in a space for selectively connecting the tube sections when the tube sections are initially separate from each other by welding the heated tube sections together and disconnecting the tube sections when the tube sections are initially joined together, the improvement being in that said lower clamp member of at least one of said holders has a groove longitudinally across its upper surface for locating its tube section on said lower clamp member, and a recess extending transversely outwardly from said groove to receive a key secured to the tube section for accurately locating the tube section in said lower clamp member.
2. The device wherein said recess extends completely across said groove on both sides of said groove.
3. The device in combination with a plastic tube section, said plastic tube section having a key fixed thereto, and said key being mounted in said recess.
4. The device wherein said tube section terminates in a flat seal, and said key locating said flat seal parallel to the upper surface of said lower clamp member.
5. The device wherein said tube section having said key is connected to a solution bag carrying a fluid therein, and a clamp member mounted to said tube section between said key and said solution bag for creating a dry chamber in said tube section between said clamp member and said seal.
6. The device wherein said key includes an upper wall flush with said upper surface of said lower clamp member.
7. The device wherein said key includes a tube enclosing member mounted around said tube section, and said upper wall of said key extending across and outwardly of said tube enclosing member.
8. The device wherein said tube enclosing member includes an inner wall having a circular opening therethrough, said tube section being formed from two separate tube portions, each of said tube portions being telescoped into said tube enclosing member and abutting against said inner wall with each of said tube portions being on an opposite side of said inner wall, and said tube portions having an inner surface flush with the inner surface formed by said circular opening in said inner wall to create an unobstructed flow path through said tube portions and said key.
9. The device wherein at least portions of said tube enclosing member fits snugly against and contacts said recess.
10. A tube assembly for use with a sterile connect/disconnect device comprising a hollow plastic tube having two ends, a key mounted to said tube inwardly of said ends, said key being fixedly mounted to prevent rotational and sliding movement of said key with respect to said tube, said key including a tube enclosing member around said tube, and a flat wall connected to said tube enclosing member and extending outwardly thereof.
11. The tube assembly wherein said key includes a tube enclosing member mounted around said tube section, and said upper wall of said key extending across and outwardly of said tube enclosing member.
12. The tube assembly wherein said tube enclosing member includes an inner wall having a circular opening therethrough, said tube section being formed from two separate tube portions, each of said tube portions being telescoped into said tube enclosing member and abutting against said inner wall with each of said tube portions being on an opposite side of said inner wall, and said tube portions having an inner surface flush with the inner surface formed by said circular opening in said inner wall to create an unobstructed flow path through said tube portions and said key.
13. The tube assembly wherein said tube section having said key is connected to a solution bag carrying a fluid therein, and a clamp member mounted to said tube section between said key and said solution bag for creating a dry chamber in said tube section between said clamp member and said seal.
14. The tube assembly wherein said end of said tube terminates in a flat seal parallel to said flat wall of said key.
15. In a method of sterile selective connecting and disconnecting of plastic tubing wherein a first tube section is mounted between upper and lower clamping members in a first holder located side by side and spaced from a second holder with a second tube section mounted between upper and lower clamping members of the second holder, the first and the second tube sections being aligned with each other and being selectively connected or disconnected with the aid of a heated wafer passing through the space between the holders, the improvement being in mounting a key to one of the tube sections, and disposing the key in a correspondingly shaped recess in the lower clamping member of one of the holders to fix the location of that tube section in the holder.
16. The method including mounting said key in said recess with the upper surface of the key being coplanar with the upper surface of the lower clamping member.
17. The method wherein the tube section is formed of two tube portions mounting the tube portions to the key by inserting each portion into a tube enclosing member of the key until the tube portion contacts an inner wall of the tube enclosing member, and sealing the tube portions to the key.
18. The method wherein the method is used for disconnecting a plastic tubing formed by welding the first and second tube sections together, and disconnecting the first and second tube sections from each other through the location of the weld.
19. The method wherein the tube section having the key is connected to a solution bag having fluid therein clamping a portion of the tube section at a location between the solution bag and the key to create a dry chamber within the tube section between the clamp and the sealed end of the tube section connecting the tube section to another tube section, and then removing the clamp.

## Claims

1. In a device for the sterile connect/disconnect of plastic tubing having a pair of side by side tube holders, each of said tube holders having a lower clamp member and an upper clamp member movably connected to said lower clamp member for selective movement toward and away from said lower clamp member to selectively clamp a tube section therebetween with the tube section in each of said holders aligned with each other and extending into a space between said holders, and means for heating the tubes in a space for selectively connecting the tube sections when the tube sections are initially separate from each other by welding the heated tube sections together and disconnecting the tube sections when the tube sections are initially joined together, the improvement being in that said lower clamp member of at least one of said holders has a groove longitudinally across its upper surface for locating its tube section on said lower clamp member, and a recess extending transversely outwardly from said groove to receive a key secured to the tube section for accurately locating the tube section in said lower clamp member.

2. The device of Claim 1 wherein said recess extends completely across said groove on both sides of said groove.

3. The device of Claim 2 in combination with a plastic tube section, said plastic tube section having a key fixed thereto, and said key being mounted in said recess.

4. The device of Claim 3 wherein said tube section terminates in a flat seal, and said key locating said flat seal parallel to the upper surface of said lower clamp member,
wherein preferably said tube section having said key is connected to a solution bag carrying a fluid therein, and a clamp member mounted to said tube section between said key and said solution bag for creating a dry chamber in said tube section between said clamp member and said seal, and
wherein preferably said key includes an upper wall flush with said upper surface of said lower clamp member.

5. The device of any of claims 1-3 wherein said key includes a tube enclosing member mounted around said tube section, and said upper wall of said key extending across and outwardly of said tube enclosing member,
wherein preferably said tube enclosing member includes an inner wall having a circular opening therethrough, said tube section being formed from two separate tube portions, each of said tube portions being telescoped into said tube enclosing member and abutting against said inner wall with each of said tube portions being on an opposite side of said inner wall, and said tube portions having an inner surface flush with the inner surface formed by said circular opening in said inner wall to create an unobstructed flow path through said tube portions and said key,
wherein preferably at least portions of said tube enclosing member fits snugly against and contacts said recess.

6. A tube assembly for use with a sterile connect/disconnect device comprising a hollow plastic tube having two ends, a key mounted to said tube inwardly of said ends, said key being fixedly mounted to prevent rotational and sliding movement of said key with respect to said tube, said key including a tube enclosing member around said tube, and a flat wall connected to said tube enclosing member and extending outwardly thereof.

7. The tube assembly of Claim 6 wherein said key includes a tube enclosing member mounted around said tube section, and said upper wall of said key extending across and outwardly of said tube enclosing member,
wherein preferably said tube enclosing member includes an inner wall having a circular opening therethrough, said tube section being formed from two separate tube portions, each of said tube portions being telescoped into said tube enclosing member and abutting against said inner wall with each of said tube portions being on an opposite side of said inner wall, and said tube portions having an inner surface flush with the inner surface formed by said circular opening in said inner wall to create an unobstructed flow path through said tube portions and said key,
wherein preferably said tube section having said key is connected to a solution bag carrying a fluid therein, and a clamp member mounted to said tube section between said key and said solution bag for creating a dry chamber in said tube section between said clamp member and said seal ,and
wherein preferably said end of said tube terminates in a flat seal parallel to said flat wall of said key.

8. In a method of sterile selective connecting and disconnecting of plastic tubing wherein a first tube section is mounted between upper and lower clamping members in a first holder located side by side and spaced from a second holder with a second tube section mounted between upper and lower clamping members of the second holder, the first and the second tube sections being aligned with each other and being selectively connected or disconnected with the aid of a heated wafer passing through the space between the holders, the improvement being in mounting a key to one of the tube sections, and disposing the key in a correspondingly shaped recess in the lower clamping member of one of the holders to fix the location of that tube section in the holder.

9. The method of Claim 8 including mounting said key in said recess with the upper surface of the key being coplanar with the upper surface of the lower clamping member,
wherein preferably the tube section is formed of two tube portions mounting the tube portions to the key by inserting each portion into a tube enclosing member of the key until the tube portion contacts an inner wall of the tube enclosing member, and sealing the tube portions to the key,
wherein the method is preferably used for disconnecting a plastic tubing formed by welding the first and second tube sections together, and disconnecting the first and second tube sections from each other through the location of the weld, and
wherein preferably the tube section having the key is connected to a solution bag having fluid therein clamping a portion of the tube section at a location between the solution bag and the key to create a dry chamber within the tube section between the clamp and the sealed end of the tube section connecting the tube section to another tube section, and then removing the clamp.

10. A device for the sterile connect/disconnect of plastic tubing having tube holders, each of said tube holders having a lower clamp member and an upper clamp member, wherein said lower clamp member of at least one of said holders has a groove for locating its tube section, and a recess to receive a key secured to the tube section for accurately locating the tube section in said lower clamp member.
